# FASCICULE DE BREVET EUROPEEN

(11) **EP 2 271 311 B1**
(45) Date de publication et mention de la délivrance du brevet: **01.11.2017**
(21) Numéro de dépôt: 09746021.6
(22) Date de dépôt: 30.04.2009
(51) Int. Cl.: A61K 8/97, A61K 8/73, A61Q 19/00

(54) **UTILISATION COSMETIQUE D'UN ACTIF ISSU D'OPHIOPOGON JAPONICUS**
KOSMETISCHE ANWENDUNG EINES OPHIOPOGON JAPONICUS-WIRKUNGSPRINZIPS
COSMETIC USE OF AN OPHIOPOGON JAPONICUS ACTIVE PRINCIPLE

(30) Priorité: 30.04.2008 FR 0852941
(43) Date de publication de la demande: 12.01.2011
(73) Titulaire: Société Industrielle Limousine d'Application Biologique Dite SILAB, 19130 Objat (FR)
(72) Inventeur: PAUFIQUE, Jean, F-19130 Objat (FR)
(74) Mandataire: Aquinov
(86) Numéro de dépôt international: PCT/FR2009/050802
(87) Numéro de publication internationale: WO 2009/138702

(56) Documents cités:
- FR-A- 2 814 676
- DATABASE WPI Week 198720 Thomson Scientific, London, GB; AN 1987-140902 XP002511306 & JP 62 081305 A (ICHIMARU PHARCOS INC) 14 avril 1987 (1987-04-14)
- DATABASE WPI Week 200408 Thomson Scientific, London, GB; AN 2004-075316 XP002511307 & JP 2003 277285 A (NIPPON OILS & FATS CO LTD) 2 octobre 2003 (2003-10-02)
- DATABASE WPI Week 200709 Thomson Scientific, London, GB; AN 2007-086997 XP002511568 & JP 2006 347911 A (OGAWA KORYO KK) 28 décembre 2006 (2006-12-28)
- DATABASE CAPLUS [Online] CHEMICAL ABSTRACTS SERVICE, COLUMBUS, OHIO, US; 12 mai 1984 (1984-05-12), XP002511567 extrait de STN Database accession no. 1973:402722 & CHEM. & PHARM. BULLETIN, vol. 21, no. 3, 1973, pages 659-62,
- DATABASE WPI Week 200548 Thomson Scientific, London, GB; AN 2005-474609 XP002511308 & KR 2005 014 947 A (STC INT JH) 21 février 2005 (2005-02-21)

## Description

La présente invention concerne l'utilisation cosmétique d'un extrait issu d'Ophiopogon japonicus pour améliorer et/ou renforcer la fonction barrière de la peau, ainsi qu'à un procédé cosmétique de soin de la peau destiné à améliorer et/ou renforcer la fonction barrière cutanée.

L'invention se rapporte également à un principe actif issu d'Ophiopogon japonicus, à son procédé d'obtention, ainsi qu'aux compositions destinées à améliorer et/ou renforcer la fonction barrière de la peau comprenant au moins un principe actif issu d'Ophiopogon japonicus.

On sait qu'une bonne hydratation et un maintien des propriétés physiques du tissu cutané sont déterminants pour l'aspect de la peau. Dans ce cadre, des extraits de carbohydrates issu de Ophiopgon japonicus sont connus et ils ont été utilisés aux fins de l'hydratation de la peau (JP 62081305 et JP 2003277285, notamment). Cependant, les caractéristiques d'une bonne hydratation et d'un maintien des propriétés physiques de la peau sont directement liées à la cohésion des différentes couches épidermiques, en particulier du Stratum corneum et du Stratum granulosum, qui est primordiale pour assurer l'homéostasie de la fonction barrière de la peau, limiter les pertes excessives en eau et garantir une bonne hydratation cutanée.

C'est pourquoi la présente invention propose un principe actif cosmétique capable d'améliorer et/ou renforcer la fonction barrière de la peau.

En particulier la présente invention vise l'utilisation cosmétique d'un extrait issu d'Ophiopogon japonicus en tant que principe actif dans une composition cosmétique, pour améliorer et/ou renforcer la fonction barrière de la peau.

On sait que les jonctions intercellulaires, notamment les jonctions serrées, généralement appelées « tight junctions », jouent un rôle dans l'homéostasie de la fonction barrière de la peau.

Les jonctions serrées sont des complexes jonctionnels intercellulaires spécialisés qui assurent l'adhésion entre les kératinocytes du Stratum granulosum. Elles forment une barrière semi-perméable sélective et contribuent ainsi au maintien de l'hydratation des différentes couches épidermiques.

Les jonctions serrées sont constituées de 3 catégories de protéines :
- des protéines transmembranaires : l'occludine, les claudines et les molécules d'adhésion jonctionnelles (JAMs), qui jouent un rôle structural en connectant les membranes des cellules adjacentes et assurent l'adhésion cellule-cellule et l'étanchéité de la jonction serrée,
- des protéines plaques ou d'échafaudage, dont les zonula occludens (notamment ZO-1), qui lient les protéines transmembranaires aux fibres d'actine du cytosquelette de la cellule et qui recrutent les protéines de signalisation,
- des protéines de signalisation associées aux jonctions serrées qui sont impliquées dans la régulation de différents processus cellulaires comme la polarité, la prolifération et la différentiation cellulaires.

Un dysfonctionnement des jonctions serrées induit une perméabilité accrue de celles-ci et donc une augmentation du flux d'eau vers la surface cutanée. Ceci peut entraîner une altération du gradient ionique de l'épiderme qui influe largement sur les différents processus de la différenciation épidermique comme la synthèse et la maturation des lipides et des protéines impliquées dans le maintien de la fonction barrière de la peau.

C'est pourquoi, pour répondre à son objectif, la présente invention se propose d'utiliser un extrait d'Ophiopogon japonicus, tel que décrit dans les revendications, en tant que principe actif agissant préférentiellement en augmentant la formation des jonctions serrées, en particulier par stimulation de la synthèse de claudine-1 et de ZO-1.

Par ailleurs, le Stratum corneum ou couche cornée, grâce à sa structure particulière, assure la cohésion du tissu et empêche la perte excessive en eau et en solutés. La couche cornée contient un ensemble de substances hydrophiles intracornéocytaires, les NMFs (Natural Moisturizing Factors ou Facteurs naturels d'hydratation). Les NMFs, essentiellement composés d'acides aminés, d'urée, de pyrrolidone carboxylic acid (PCA) et de lactate, provenant de la dégradation de la filaggrine, sont dotés de propriétés hautement hygroscopiques et agissent comme de véritables humectants biologiques.

Leur présence permet de capter l'eau libre, de la retenir au sein des cornéocytes et de jouer ainsi un rôle important dans le maintien des propriétés physiques de la couche cornée. De plus, le lactate permet de maintenir le pH acide de la couche cornée garantissant son intégrité et sa cohésion.

En retenant l'eau dans la couche cornée et en régulant le pH, les NMFs conditionnent en partie l'homéostasie de la fonction barrière.

Or, un certain nombre de facteurs courants tels que l'âge, le vieillissement photo-induit, le nettoyage quotidien de la peau avec un savon agressif, induisent une réduction du taux de NMFs au sein du Stratum corneum, qui se traduit par une peau sèche.

Ainsi, la présente invention propose également d'utiliser un extrait d'Ophiopogon japonicus tel que décrit dans les revendications, en tant que principe actif agissant préférentiellement en augmentant le taux de NMFs de la couche cornée, en particulier en augmentant le taux de lactate et de sérine.

La présente invention vise donc l'utilisation d'un extrait issu d'Ophiopogon japonicus tel que décrit dans les revendications, en tant que principe actif dans une composition cosmétique pour améliorer et/ou renforcer la fonction barrière de la peau, ledit principe actif agissant préférentiellement en stimulant :
- la formation des jonctions serrées, et/ou
- l'augmentation du taux de NMFs de la couche cornée.
Il s'agit d'un principe actif issu d'Ophiopogon japonicus riche en fructosanes.

La présente invention permet ainsi d'assurer la cohésion des couches supérieures de l'épiderme.

Avantageusement, elle permet de diminuer la perte insensible en eau de la peau et de réduire la desquamation de la couche cornée.

Ainsi, en renforçant la fonction barrière de la peau et en restructurant l'épiderme, le principe actif extrait d'Ophiopogon japonicus selon l'invention, a un effet cosmétique hydratant et restructurant de la peau.

En particulier, l'extrait d'Ophiopogon japonicus selon l'invention peut être utilisé en tant que principe actif dans une composition à visée hydratante. L'invention vise un extrait d'Ophiopogon japonicus, particulièrement adapté pour une utilisation en tant que principe actif dans une composition cosmétique, pour améliorer et/ou renforcer la fonction barrière de la peau. Il s'agit d'un extrait issu d'Ophiopogon japonicus, comprenant au moins 60% de fructosanes (en poids par rapport au poids total de l'extrait sec) et obtenu par un procédé comprenant au moins une étape d'hydrolyse enzymatique des carbohydrates. L'invention concerne également un procédé d'obtention particulier d'un tel extrait.

L'invention vise aussi une composition cosmétique destinée à améliorer et/ou renforcer la fonction barrière de la peau, comprenant ledit extrait d'Ophiopogon japonicus en tant que principe actif.

La composition selon l'invention peut contenir de 0,01 à 20 % dudit extrait d'Ophiopogon japonicus en tant que principe actif.

L'administration d'une telle composition est de préférence effectuée par voie topique.

La composition selon l'invention peut se présenter sous forme de crèmes, émulsions huile-dans-eau, eau-dans-huile ou émulsions multiples, solutions, suspensions ou encore poudres.

Enfin, l'invention a également pour objet un procédé cosmétique de soin de la peau, destiné à renforcer et/ou améliorer la fonction barrière de la peau, comprenant l'application topique sur la peau d'une composition renfermant ledit principe actif issu de d'Ophiopogon japonicus.

La présente invention est maintenant décrite en détail.

### 1/ PROCEDE D'OBTENTION D'UN PRINCIPE ACTIF SELON L'INVENTION:

Un principe actif issu d'Ophiopogon japonicus utile selon l'invention, est susceptible d'être obtenu par un procédé comprenant au moins une étape d'hydrolyse enzymatique.

En particulier, le principe actif issu d'Ophiopogon japonicus utile selon l'invention, est susceptible d'être obtenu par un procédé comprenant les étapes suivantes :
- solubilisation de poudre de tubercules d'Ophiopogon japonicus dans l'eau,
- au moins une hydrolyse enzymatique,
- séparation des phases soluble et insoluble, et
- inactivation enzymatique par traitement thermique.

Ces étapes sont usuelles dans le domaine des extractions d'actifs à partir de plantes, et l'homme du métier est à même d'en ajuster les paramètres réactionnels sur la base de ses connaissances générales.

L'inactivation enzymatique peut être suivie d'une ou plusieurs étapes de filtration(s) et/ou de concentration(s). Préférentiellement, l'inactivation enzymatique est suivie par au moins une étape de filtration et une étape de filtration stérilisante.

Les enzymes utilisées sont des carbohydrases. Il peut s'agir d'enzymes purifiées, d'enzymes mono-constituant ou encore d'un mélange de différentes enzymes.

Le principe actif peut être obtenu sous forme liquide ou sous forme de poudre par atomisation ou lyophilisation.

### 2/ CARACTERISATION D'UN PRINCIPE ACTIF SELON L'INVENTION :

Un principe actif particulièrement adapté selon l'invention est un extrait issu d'Ophiopogon japonicus comprenant au moins 60% de fructosanes en poids par rapport au poids total de matière sèche, préférentiellement au moins 80%.

La présence de fructosanes dans le principe actif, en particulier une teneur d'au moins 60% du poids total de la matière sèche, confère notamment son activité spécifique au principe actif selon l'invention.

Selon un mode de réalisation préféré, il s'agit d'un extrait issu de tubercules d'Ophiopogon japonicus.

Le principe actif selon l'invention peut être défini par les caractéristiques exposées ci-après.

### 2-1/ Matières sèches

On mesure le taux de matières sèches par passage à l'étuve à 105°C en présence de sable d'un échantillon de poids initial donné jusqu'à obtention d'un poids constant.

Le taux de matières sèches d'un principe actif issu d'Ophiopogon japonicus selon l'invention est compris entre 30 et 250 g/l, plus particulièrement entre 90 et 130 g/l.

### 2-2/ Mesure du pH

Le pH mesuré par la méthode potentiométrique à température ambiante conduit à des valeurs comprises entre 2,5 et 6,5, plus particulièrement entre 3,0 et 4,0.

### 2-3/ Détermination de la teneur en fructose

Le fructose est dosé par spectrophotométrie selon la méthode de détermination des cétoses (Boratynski J., (1984), Analytical Biochemistry, 137, 2, 528-532)

Le taux de fructose d'un principe actif issu de d'Ophiopogon japonicus selon l'invention est compris entre 28 et 245 g/l, préférentiellement entre 85 et 127 g/l.

### 2-4/ Caractérisation des carbohydrates

La détermination de la taille des carbohydrates d'un principe actif issu d'Ophiopogon japonicus selon l'invention est réalisée par chromatographie liquide haute performance.

Le chromatogramme obtenu montre la présence d'environ 30% de monosaccharides de masse moléculaire inférieure à 180Da et d'environ 70% d'oligosaccharides et polysaccharides de masse molaire comprise entre 180 et 7500Da.

La fraction glucidique du principe actif issu d'Ophiopogon japonicus selon l'invention est donc composée essentiellement d'oligosaccharides et de polysaccharides.

### 2-5/ Identification de la fraction active

La fraction active du principe actif issu d'Ophiopogon japonicus, agissant sur la synthèse de ZO-1, est constituée majoritairement par des molécules de poids moléculaire supérieur à 500Da. Elle est composée par des oligofructosanes et polyfructosanes.

### 3. EVALUATION DE L'EFFET D'UN PRINCIPE ACTIF ISSU D'OPHIOPOGON JAPONICUS :

Le principe actif utilisé pour les études qui suivent est un extrait issu d'Ophiopogon japonicus, obtenu par la mise en oeuvre d'un procédé comprenant les étapes suivantes :
- solubilisation de poudre de tubercules d'Ophiopogon japonicus dans l'eau à raison d'au moins 50g/l,
- hydrolyses enzymatiques des carbohydrates,
- séparation des phases soluble et insoluble par décantation,
- inactivation enzymatique par traitement thermique,
- filtration et filtration stérilisante sur un filtre de 0,22µm.

Le principe actif obtenu présente les caractéristiques suivantes :
- aspect : liquide limpide
- couleur : jaune
- matières sèches : 128,3g/l
- fructose : 114,9g/l (essentiellement sous forme d'oligofructosanes et polyfructosanes),
- pH : 3,3.

Cet exemple d'extrait n'est bien entendu pas limitatif.

### 3.1 - Effet d'un principe actif issu d'Ophiopogon japonicus sur la synthèse de claudine-1 et de ZO-1

Cette étude a pour objectif d'évaluer l'effet d'un principe actif issu d'Ophiopogon japonicus selon l'invention sur la synthèse de claudine-1 et de Zonula Occudens (ZO-1), deux protéines majeures des jonctions serrées qui sont très impliquées dans la cohésion cellule-cellule et l'homéostasie de la fonction barrière.

L'étude est réalisée par Western Blot sur kératinocytes humains normaux. Le protocole opératoire est le suivant.

A J1, les kératinocytes sont ensemencés en milieu complet et incubés à 37°C.

A J5, le milieu de culture est éliminé et remplacé par du milieu contenant un principe actif selon l'invention à 0,5% et 1% ou du CaCl₂ utilisé comme témoin positif. Un témoin non traité est également réalisé.

Les cellules sont à nouveau incubées à 37°C.

A J6 les extraits cellulaires sont récupérés.

Des Western-Blot sont ensuite réalisés pour doser la claudine-1 et le ZO-1.

Les résultats obtenus sont présentés dans le tableau suivant :

| | Synthèse (%) | |
|---|---|---|
| | Claudine-1 | ZO-1 |
| Témoin non traité | 100 | 100 |
| CaCl₂ | 148 | 124 |
| Principe actif issu d'Ophiopogon japonicus à 0,5% | 123 | 118 |
| Principe actif issu d'Ophiopogon japonicus à 1% | 126 | 127 |

On constate que testé à 1%, un principe actif issu d'Ophiopogon japonicus selon l'invention augmente significativement la synthèse de claudine-1 de 26% et de ZO-1 de 27% par des kératinocytes humains normaux, et favorise ainsi la formation des jonctions serrées.

### 3.2 - Effet sur le réseau membranaire de ZO-1

L'objectif de cette étude est de visualiser l'effet d'un principe actif issu d'Ophiopogon japonicus sur le réseau membranaire formé par ZO-1, un des constituants majeurs des jonctions serrées.

L'étude est réalisée par immunocytologie sur des kératinocytes humains normaux après agression au Sodium Lauryl Sulfate (SLS).

Le protocole opératoire est décrit en suivant.

A J1, les kératinocytes humains sont ensemencés sur des lames de verre dans du milieu de culture complet.

A J4, les kératinocytes sont traités avec une solution de CaCl₂ pour permettre la formation d'un réseau de jonctions serrées.

A J6, les cellules sont traitées avec une solution de SLS. A la fin de l'incubation la solution de SLS est éliminée et remplacée par un milieu contenant 1% d'un principe actif issu d'Ophiopogon japonicus selon l'invention. Les cellules sont ensuite incubées à 37°C.

A J8, on réalise un immunomarquage avec des anticorps anti-ZO-1. On visualise ensuite l'intensité du marquage sur un microscope couplé à un système d'analyse d'images.

Les résultats immunocytologiques étant qualitatifs, quatre niveaux d'expression ont été définis :
- absence de détection d'immunoréactivité -
- faible détection d'immunoréactivité +
- moyenne détection d'immunoréactivité ++
- forte détection d'immunoréactivité +++

Les résultats obtenus sont présentés dans le tableau ci-dessous :

| | Expression des ZO-1 |
|---|---|
| Témoin non agressé | +++ |
| Témoin agressé SLS | - |
| Témoin agressé SLS + traité avec un extrait d d'Ophiopogon japonicus selon l'invention à 1% | +++ |

Après une agression SLS sur kératinocytes humains, on constate que le réseau de ZO-1 membranaire induit par le CaCl₂ est fortement altéré. Ces résultats montrent que testé à 1%, un principe actif issu d'Ophiopogon japonicus selon l'invention, restaure la synthèse de ZO-1 et la formation du réseau membranaire de ZO-1 des kératinocytes humains après agression SLS.

### 3.3 Evaluation de l'effet hydratant

### 3.3.1 Effet sur le taux de NMFs du Stratum corneum

L'objectif de cette étude est d'évaluer in vivo l'influence d'un principe actif issu d'Ophiopogon japonicus formulé à 3% en gel sur les NMFs (Natural Moisturizing Factors ou Facteurs naturels d'hydratation).

Cette étude est réalisée par dosage du lactate et de la sérine, composants des NMFs.

Le dosage du lactate est réalisé au niveau des mollets de 20 volontaires sains de sexe féminin présentant une peau sèche (taux d'hydratation inférieur à 50).

Le dosage de la sérine est réalisé au niveau des mollets sur 14 volontaires sains de sexe féminin présentant une peau sèche (taux d'hydratation inférieur à 50).

Le protocole opératoire est le suivant.

Entre J-7 et J0, les volontaires arrêtent toute application de crème au niveau des mollets.

A J0, on détermine pour chaque volontaire 3 zones au niveau des mollets:
- une zone non traitée,
- une zone placebo, et
- une zone traitée avec un actif issu d'Ophiopogon japonicus formulé à 3%.

On prélève sur chacune de ces zones des échantillons de couche cornée.

Entre J0 et J13, les volontaires appliquent sur leurs mollets deux fois par jour le placebo ou l'actif issu d'Ophiopogon japonicus formulé à 3%.

A J14, on détermine à nouveau les trois zones des mollets et on réalise des prélèvements sur chaque zone.

Sur chaque échantillon prélevé, des dosages du lactate et de la sérine sont réalisés.

Les résultats obtenus sont présentés dans le tableau ci-dessous en pourcentage de variation par rapport au placebo:

| | Variation / Placebo (%) |
|---|---|
| Taux de lactate | +41 |
| Taux de sérine | +20 |

Dans les conditions de cette étude, on constate qu'après 14 jours d'applications biquotidiennes, en comparaison au placebo, un principe actif issu d'Ophiopogon japonicus formulé à 3% en gel, améliore significativement la cohésion du stratum corneum en augmentant la concentration en lactate de la peau de 41% et la concentration en sérine de 20%.

### 3.3.2 Etude du pouvoir hydratant long terme

Cette étude a pour but de quantifier in vivo l'effet hydratant d'un principe actif issu d'Ophiopogon japonicus formulé à 3% en gel.

L'étude est réalisée sur 25 volontaires sains de sexe féminin présentant une peau sèche au niveau des mollets (taux d'hydratation inférieur à 50).

Les mesures sont réalisées à l'aide d'un cornéomètre muni d'une sonde qui mesure de façon quantitative et directe la capacitance électrique de la peau. Cette mesure est reliée directement à l'état d'hydratation de la peau.

Entre J-7 et J0, les volontaires arrêtent toute application de crème au niveau des mollets.

A J0, on détermine pour chaque volontaire 3 zones au niveau des mollets:
- une zone non traitée,
- une zone placebo, et
- une zone traitée avec un actif issu d'Ophiopogon japonicus formulé à 3%.

On mesure sur chaque zone le taux d'hydratation.

Entre J0 et J13, les volontaires appliquent sur leurs mollets deux fois par jour le placebo ou l'actif issu d'Ophiopogon japonicus formulé à 3%.

A J14, on détermine à nouveau les trois zones des mollets et on mesure le taux d'hydratation.

Les résultats obtenus montrent que le taux d'hydratation pour les zones traitées avec l'actif issu d'Ophiopogon japonicus formulé à 3%, augmente de 13% par rapport au taux d'hydratation des zones traitées avec le placebo.

Ainsi, dans les conditions de cette étude, après 14 jours d'applications biquotidiennes et en comparaison au placebo, un principe actif issu d'Ophiopogon japonicus selon l'invention, augmente de 13% le taux d'hydratation.

### 3.4 Evaluation de l'effet sur la restructuration du Stratum corneum

### 3.4.1 Effet sur la perte insensible en eau

L'objectif de cette étude est de quantifier in vivo l'effet d'un principe actif issu d'Ophiopogon japonicus formulé à 3% en gel sur la perte insensible en eau de la peau.

Lorsque la barrière cutanée est lésée, il apparaît des dérèglements dans la régulation des échanges d'eau : l'eau migre plus facilement ce qui augmente la perte insensible en eau.

L'étude est réalisée sur 20 volontaires sains de sexe féminin présentant une peau normale au niveau des bras.

Les mesures de perte insensible en eau sont effectuées avec un Tewamètre muni d'une sonde qui mesure le gradient de vapeur d'eau se mettant en place entre la surface cutanée et l'air ambiant. Cette mesure donne une information sur la qualité de la fonction barrière de la peau.

Le protocole de l'étude est décrit ci-après.

Entre J-7 et J0, les volontaires n'appliquent aucune crème au niveau des bras.

A J0, on détermine trois zones de mesure au niveau du haut des bras :
- une zone non traitée,
- une zone placebo,
- une zone traitée avec un principe actif issu d'Ophiopogon japonicus selon l'invention formulé à 3% en gel. Une mesure au Tewamètre est effectuée sur chaque zone. Entre J0 et J13, les volontaires :
- lavent les zones étudiées avec un savon irritant SLS (Sodium Lauryl Sulfate). Le SLS permet d'augmenter les pertes en eau et d'amplifier l'effet restructurant et la fonction barrière de la couche cornée, et
- appliquent deux fois par jour le placebo et le principe actif issu d'Ophiopogon japonicus formulé à 3% en gel.

A J14, on réalise sur chaque zone une mesure au Tewamètre.

Les résultats obtenus montrent que la perte insensible en eau pour les zones traitées avec l'actif issu d'Ophiopogon japonicus formulé à 3%, diminue de 11% par rapport à la perte insensible en eau des zones traitées avec le placebo.

Ainsi, dans les conditions de cette étude, après 14 jours d'applications biquotidiennes et en comparaison au placebo, un principe actif issu d'Ophiopogon japonicus selon l'invention, diminue de 11% la perte insensible en eau après agressions répétées au SLS. Le principe actif issu d'Ophiopogon japonicus limite donc l'altération de la fonction barrière et préserve ainsi l'intégrité du Stratum corneum.

### 3.4.2 Effet sur la cohésion du Stratum corneum

Cette étude a pour but de quantifier in vivo l'effet d'un principe actif issu d'Ophiopogon japonicus selon l'invention formulé à 3% en gel, sur la cohésion du Stratum corneum en mesurant un indice de desquamation et la surface totale occupée par les squames.

La desquamation de la peau est une des résultantes de la perte d'intégrité de la couche cornée.

L'étude est réalisée sur 25 volontaires sains de sexe féminin présentant une peau sèche au niveau des mollets (taux d'hydratation inférieur à 50).

L'étude de la cohésion du Stratum corneum est réalisée à l'aide d'un profilomètre muni d'un analyseur d'images qui permet d'obtenir les paramètres suivants :
- l'indice de desquamation, et
- la surface totale occupée par les squames.

Le protocole opératoire est indiqué en suivant.

Entre J-7 et J0, les volontaires n'appliquent aucune crème au niveau des mollets.

A J0, on détermine deux zones de mesure au niveau des mollets :
- une zone placebo,
- une zone traitée avec un principe actif issu d'Ophiopogon japonicus selon l'invention formulé à 3% en gel. On réalise des prélèvements sur chaque zone étudiée à l'aide d'un D-squame.

Entre J0 et J13, les volontaires appliquent deux fois par jour le placebo et le principe actif issu d'Ophiopogon japonicus formulé à 3% en gel.

A J14, on détermine les zones au niveau des mollets et on réalise des prélèvements sur chaque zone.

Les résultats obtenus sont présentés dans le tableau ci-dessous en pourcentage de variation par rapport au placebo :

| | Variation / Placebo (%) |
|---|---|
| Indice de desquamation | -25 |
| Surface occupée par les squames | -27 |

Dans les conditions de cette étude, on constate qu'après 14 jours d'applications biquotidiennes, en comparaison au placebo, un principe actif issu d'Ophiopogon japonicus formulé à 3% en gel, entraîne une diminution de 25% de l'indice de desquamation et de 27% de la surface occupée par les squames. Le principe actif issu d'Ophiopogon japonicus assure ainsi la cohésion de la couche cornée en limitant la desquamation excessive.

### 4. EXEMPLES DE COMPOSITION

La présente invention couvre aussi les compositions cosmétiques et/ou dermopharmaceutiques incluant au moins un principe actif issu d'Ophiopogon japonicus selon la présente invention dans différentes formes galéniques, adaptées à l'administration par voie topique cutanée.

Ces compositions peuvent se présenter notamment sous forme de crèmes, émulsions huile-dans-eau, émulsions eau-dans-huile, émulsions multiples, solutions, suspensions ou poudres. Elles peuvent être plus ou moins fluides et avoir l'aspect d'une crème, d'une lotion, d'un lait, d'un sérum, d'une pommade, d'un gel, d'une pâte ou d'une mousse, ou sous forme solide.

Ces compositions contiennent entre 0,01 et 20% en poids de principe(s) actif(s) issu(s) d'Ophiopogon japonicus selon la présente invention.

Ces compositions comprennent, outre l'actif, un milieu physiologiquement acceptable et de préférence cosmétiquement acceptable, c'est-à-dire qui ne provoque pas de sensations d'inconfort inacceptables pour l'utilisateur tels que des rougeurs, tiraillements ou picotements. Ce milieu contient généralement de l'eau.

Les compositions selon l'invention peuvent contenir comme adjuvant au moins un composé choisi parmi :
- les huiles, qui peuvent être choisies notamment parmi les huiles de silicone, linéaires ou cycliques, volatiles ou non volatiles ;
- les cires, telles que l'ozokérite, la cire de polyéthylène, la cire d'abeille ou la cire de carnauba ;
- les élastomères de silicone ;
- les tensioactifs, de préférence émulsionnants, qu'ils soient non ioniques, anioniques, cationiques ou amphotères ;
- les co-tensioactifs, tels que les alcools gras linéaires ;
- les épaississants et/ou gélifiants ;
- les humectants, tels que les polyols comme la glycérine ;
- les filtres organiques ;
- les filtres inorganiques ;
- les colorants, les conservateurs, les charges ;
- les tenseurs ;
- les séquestrants ;
- les parfums ;
- et leurs mélanges, sans que cette liste ne soit limitative.

Des exemples de tels adjuvants sont cités notamment dans le Dictionnaire CTFA (International Cosmetic Ingrédient Dictionary and Handbook publié par le Personal Care Product Council).

Ces compositions sont notamment destinées à renforcer et/ou améliorer la fonction barrière de la peau.

L'invention vise à cet effet un procédé cosmétique de soin de la peau humaine, destiné à renforcer et/ou améliorer la fonction barrière cutanée, comprenant l'application topique sur la peau d'une composition renfermant un principe actif issu d'Ophiopogon japonicus, en particulier une composition renfermant entre 0,01 et 20% en poids de principe(s) actif(s) particulier(s) issu(s) d'Ophiopogon japonicus selon la présente invention tels que décrits aux points 1 et 2.

On peut citer des formulations ayant montré une stabilité physique incluant 5% de principe actif selon l'invention.

### Gel limpide :

- Carbopol : 0,5% avec Triéthanolamine : qsp pH=6,5
- Glycérol : 10%
- Propylène glycol : 10%
- Conservateur : 1,0%
- Principe actif : 5,0%
- Eau : 73,5%

### Gel opaque:

- Sépigel 305 : 3%
- Lanol 99 : 12%
- Conservateur : 1,0%
- Principe actif : 5,0%
- Eau : 79%

### Gel émulsionné :

- Montanov 202 : 3,0%
- Isopropyl palmitate : 10%
- Conservateur : 1,0%
- Sépigel 305 : 2%
- Principe actif : 5,0%
- Eau : 79%

### Emulsion non ironique :

- Montane 60 : 2,0%
- Montanox 60 : 4,0%
- Isopropyl myristate : 8%
- Paraffin wax 130/135 : 3%
- Conservateur : 1,0%
- Principe actif : 5,0%
- Eau : 77%

### Emulsion anionique :

- Acide stéarique : 7,0% Triéthanolamine qsp pH=8
- Ritaphyl ICS : 20%
- Conservateur : 1,0%
- Principe actif : 5,0%
- Eau : 67%

### Emulsion cationique :

- Quaternium-82 : 5,0%
- Alcool cétylique : 1%
- Gemseal 60 : 8%
- Alcool cétéarylique : 1%
- PEG 100 stéarate : 1%
- Conservateur : 1,0%
- Principe actif : 5,0%
- Eau : 78%

De plus, des tests ont montré la compatibilité du principe actif avec les matières premières utilisées en cosmétique : épaississants, émulsionnants, solvants.

On peut également citer des exemples de compositions cosmétiques destinées à améliorer et/ou renforcer la fonction barrière de la peau, incluant le principe actif selon l'invention. Les exemples de composition qui suivent sont obtenus par mélange des différents composants. Les quantités indiquées sont données en pourcentage de poids.

### 4.1 Exemple d'un sérum pré traitant

La formulation est la suivante :

| | |
|---|---|
| - DC 345 (Dow Corning) : | 2% |
| - BRIJ 72 (Uniquema) | 1,2% |
| - DC 5200 (Dow Corning) | 2% |
| - Arlatone 2121 (ICI) | 2% |
| - BRIJ 721 (Uniquema) | 0,8% |
| - Conservateur: | 1% |
| - Principe actif selon l'invention : | 3% |
| - Eau : | qsp 100% |
| La solution a un pH de 5,5. | |

### 4.2 Exemple d'une crème de soin

La formulation est la suivante :

| | |
|---|---|
| - Montanox 60 (Seppic) | 1% |
| - DUB STG 30 AE (Stéarinerie Dubois) | 5% |
| - Diamant N (Cornelius) | 2% |
| - Montane 60 (Seppic) | 2% |
| - DC 345 (Dow corning) | 8% |
| - DUB IMP(Stéarinerie Dubois) | 2% |
| - Conservateur: | 1% |
| - Principe actif selon l'invention : | 3% |
| - Eau : | qsp 100% |
| La solution a un pH de 6,2. | |

### 4.3 Exemple d'un lait hydratant épais

La formulation est la suivante :

| | |
|---|---|
| - DUB Diol (Stéarinerie Dubois) | 5% |
| - Stéarine P200 micronisée (Stéarinerie Dubois) | 1,2% |
| - Cethyl Alcohol (Stéarinerie Dubois) | 1% |
| - DC 345 (Dow Corning) | 8% |
| - DUB PTCC (stéarinerie Dubois) | 2% |
| - Gemseal 40 (Total) | 2% |
| - Montanov 82 (Seppic) | 2% |
| - Conservateur: | 1% |
| - Principe actif selon l'invention : | 3% |
| - Eau : | qsp 100% |
| La solution a un pH de 5,3. | |

## Revendications

1. Principe actif, adapté à une utilisation cosmétique dans une composition cosmétique pour améliorer et/ou renforcer la fonction barrière de la peau, **caractérisé en ce qu'**il s'agit d'un extrait issu *d'Ophiopogon japonicus* obtenu par un procédé comprenant au moins une étape d'hydrolyse enzymatique **des carbohydrates,** ledit extrait comprenant au moins 60% de fructosanes en poids total de matière sèche.

2. Principe actif selon la revendication 1, **caractérisé en ce qu'**il s'agit d'un extrait de tubercules *d'Ophiopogon japonicus.*

3. Principe actif selon l'une des revendications 1 ou 2, **caractérisé par** :
- un taux de matières sèches compris entre 30 et 250 g/l,
- un pH compris entre 2,5 et 6,5,
- une teneur en fructose comprise entre 28 et 245 g/l.

4. Principe actif selon l'une des revendications 1 à 3, **caractérisé par** :
- un taux de matières sèches compris entre 90 et 130 g/l,
- un pH compris entre 3,0 et 4,0,
- une teneur en fructose comprise entre 85 et 127 g/l.

5. Procédé d'obtention d'un principe actif selon l'une quelconque des revendications précédentes, **caractérisé en ce qu'**il comprend les étapes suivantes :
- solubilisation de poudre de tubercules *d'Ophiopogon japonicus* dans l'eau,
- au moins une hydrolyse enzymatique des carbohydrates,
- séparation des phases soluble et insoluble,
- inactivation enzymatique par traitement thermique, et
- filtration(s).

6. Composition cosmétique destinée à améliorer et/ou renforcer la fonction barrière de la peau, contenant entre 0,01% et 20% d'au moins un principe actif selon l'une des revendications 1 à 4.

7. Composition cosmétique selon la revendication 6., **caractérisée en ce qu'**elle se présente sous forme de crème, émulsion huile-dans-eau, émulsion eau-dans-huile, émulsions multiples, solution, suspension ou poudre.

8. Procédé cosmétique de soin de la peau humaine, destiné à renforcer et/ou améliorer la fonction barrière cutanée, comprenant l'application topique sur la peau d'une composition selon l'une des revendications 6 ou 7.

## Patentansprüche

1. Wirkstoff, der zu einer kosmetischen Verwendung in einer kosmetischen Zusammensetzung eingerichtet ist, um die Barrierefunktion der Haut zu verbessern und/oder zu verstärken, **dadurch gekennzeichnet, dass** es sich um einen Extrakt handelt, der aus *Ophiopogon japonicus* mit einem Verfahren hergestellt worden ist, das wenigstens einen Verfahrensschritt einer enzymatischen Hydrolyse von Kohlenhydraten umfasst, wobei der Extrakt einen Fruktosangehalt von wenigstens 60% des Gesamtgewichts der Trockenmasse aufweist.

2. Wirkstoff nach Anspruch 1, **dadurch gekennzeichnet, dass** es sich um einen Extrakt aus den Knollen von *Ophiopogon japonicus* handelt.

3. Wirkstoff nach einem der Ansprüche 1 oder 2, **gekennzeichnet durch**:
- einen Trockenmasseanteil zwischen 30 und 250 g/l,
- einem pH-Wert zwischen 2,5 und 6,5,
- einen Fruktosegehalt zwischen 28 und 245 g/l.

4. Wirkstoff nach einem der Ansprüche 1 bis 3, **gekennzeichnet durch**:
- einen Trockenmasseanteil zwischen 90 und 130 g/l,
- einen pH-Wert zwischen 3,0 und 4,0,
- einem Fruktosegehalt zwischen 85 und 127 g/l.

5. Verfahren zur Herstellung eines Wirkstoffes nach einem der vorhergehenden Ansprüche, **dadurch gekennzeichnet, dass** dieses die folgenden Verfahrensschritte umfasst:
- Lösen von Pulver der Knollen von *Ophiopogon japonicus* in Wasser,
- wenigstens eine enzymatische Hydrolyse von Kohlenhydraten,
- Trennen der löslichen und unlöslichen Phasen,
- enzymatisches Inaktivieren durch Wärmebehandlung, und
- Filtrierung(en).

6. Kosmetische Zusammensetzung, die dazu vorgesehen ist, die Barrierefunktion der Haut zu verbessern und/oder zu verstärken, und die zwischen 0,01% und 20% von wenigstens einem Wirkstoff nach einem der Ansprüche 1 bis 4 enthält.

7. Kosmetische Zusammensetzung nach Anspruch 6, **dadurch gekennzeichnet, dass** diese in der Gestalt einer Creme, einer Emulsion von Öl in Wasser, einer Emulsion von Wasser in Öl, einer mehrfachen Emulsion, einer Lösung, einer Suspension oder als Pulver vorliegt.

8. Kosmetisches Verfahren zur Pflege der menschlichen Haut, das dazu vorgesehen ist, die Barrierefunktion der Haut zu verstärken und/oder zu verbessern, und das auf der Haut die topische Anwendung einer Zusammensetzung nach einem der Ansprüche 6 oder 7 umfasst.

## Claims

1. An active principle, suitable for cosmetic use in a cosmetic composition for improving and/or reinforcing the barrier function of the skin, **characterised in that** it comprises an extract issuing from *Ophiopogon japonicus* obtainable by a method comprising at least an enzymatic hydrolysis of carbohydrates step, said extract comprising at least 60% fructosans by total weight of dry matter.

2. An active principle according to claim 1, **characterised in that** it is an extract of *Ophiopogon japonicus* tubers.

3. An active principle according to claim 1 or 2, **characterised by**:
- a proportion of dry matter of between 30 and 250 g/l,
- a pH of between 2.5 and 6.5,
- a fructose content of between 28 and 245 g/l.

4. An active principle according to any of claims 1 to 3, **characterised by**:
- a proportion of dry matter of between 90 and 130 g/l,
- a pH of between 3.0 and 4.0,
- a fructose content of between 85 and 127 g/l.

5. A method for obtaining an active principle according to any of the preceding claims, **characterised in that** it comprises the following steps:
- solubilisation of *Ophiopogon japonicus* tuber powder in water,
- at least an enzymatic hydrolysis of carbohydrates,
- separation of the soluble and insoluble phases,
- enzyme inactivation by heat treatment, and
- filtration(s).

6. A cosmetic composition intended to improve and/or reinforce the barrier function of skin, containing between 0.01% and 20% of at least one active principle according to any of claims 1 to 4.

7. A cosmetic composition according to claim 6, **characterised in that** it is in the form of cream, oil-in-water emulsion, water-in-oil emulsion, multiple emulsions, solution, suspension or powder.

8. A cosmetic method for caring for the human skin, intended to reinforce and/or improve the skin barrier function, comprising the topical application to the skin of a composition according to claim 6 or 7.
